# EUROPEAN PATENT APPLICATION

(11) **EP 4 613 301 A1**
(43) Date of publication of application: **10.09.2025**
(21) Application number: 23885591.0
(22) Date of filing: 24.10.2023
(51) Int. Cl.: A61M 5/32

(54) **INJECTION NEEDLE**

(30) Priority: 31.10.2022 JP 2022174505
(71) Applicant: TSK Laboratory, Japan, Tochigi 328-0002 (JP)
(72) Inventor: KAWASHIMA, Ken, Tochigi-shi, Tochigi 328-0002 (JP); FUJISAWA, Toru, Tochigi-shi, Tochigi 328-0002 (JP); HIGANO, Satoshi, Tochigi-shi, Tochigi 328-0002 (JP); KOBAYASHI, Hirotsugu, Tochigi-shi, Tochigi 328-0002 (JP); SHIBATA, Ryo, Tochigi-shi, Tochigi 328-0002 (JP)
(74) Representative: Serjeants LLP
(86) International application number: PCT/JP2023/038364
(87) International publication number: WO 2024/095831

(57) **Abstract**

In order to provide an injection needle capable of reliably reducing the piercing resistance even when the injection needle has a side opening, there is provided an injection needle 10 including: a main body 12 portion having a hollow cylindrical shape; a distal end portion 14 having a curved outer shape, the distal end portion 14 being joined so as to close a distal end of the main body portion 12; and a side surface opening portion 20 opened on a side surface of the main body portion 12, wherein a region surrounding the side surface opening portion 20 has a substantially arc-shaped concave shape in a side view along an axial direction G in a state where the side surface opening portion 20 is disposed on an upper side, wherein a distal end side surface chamfered portion 30 having a gently curved surface is provided at a position corresponding to an edge on a distal end side of the concave shape, and wherein a rear end side surface chamfered portion 40 having a gently curved surface is provided at a position corresponding to an edge on a rear end side of the concave shape.

## Description

### TECHNICAL FIELD

The present invention relates to an injection needle, particularly an injection needle having an opening portion on a side surface.

### BACKGROUND ART

Although reducing piercing resistance is an important problem of the injection needle, in order to cope with this problem, an injection needle formed of a superelastic metal and having a wall thickness of 0.3 mm or less has been proposed (see, for example, Patent Document 1).

### PRIOR ART DOCUMENT

### PATENT DOCUMENT

Patent Document 1: JP-A-H6-225940

### SUMMARY OF THE INVENTION

### PROBLEMS TO BE SOLVED BY THE INVENTION

The injection needle described in Patent Literature 1 is a general injection needle having an opening at a distal end, but a so-called side hole injection needle having an opening on a side surface of the injection needle is also used in various fields including cosmetic surgery. In such a side-hole injection needle, the injection needle may be moved or rotated in the front-back direction in the skin in order to spread a medical solution widely in the skin. For this reason, the piercing resistance increases, and there is a possibility that intradermal internal bleeding or blood vessel damage occurs. Therefore, in the side-hole injection needle, it is more important to reduce the piercing resistance.

Therefore, an object of the present invention is to solve the above problems, and it is an object of the present invention to provide an injection needle capable of reliably reducing piercing resistance even when the injection needle has an opening on a side surface.

### SOLUTIONS TO THE PROBLEMS

An injection needle according to one embodiment of the present invention includes:
a main body portion having a hollow cylindrical shape;
a distal end portion having a curved outer shape, the distal end portion being joined so as to close a distal end of the main body portion; and
a side surface opening portion opened on a side surface of the main body portion,
in which a region surrounding the side surface opening portion has a substantially arc-shaped concave shape in a side view along an axial direction in a state where the side surface opening portion is disposed on an upper side,
in which a distal end side surface chamfered portion having a gently curved surface is provided at a position corresponding to an edge on a distal end side of the concave shape, and
in which a rear end side surface chamfered portion having a gently curved surface is provided at a position corresponding to an edge on a rear end side of the concave shape.

### EFFECTS OF THE INVENTION

According to the present invention, it is possible to provide an injection needle capable of reliably reducing the piercing resistance even when the injection needle has a side opening.

### BRIEF DESCRIPTION OF THE DRAWINGS

Fig. 1 is a perspective view schematically showing an injection needle according to one embodiment of the present invention.
Fig. 2 is a perspective view schematically showing a conventional injection needle having a side opening.
Fig. 3 is a view showing a general method for manufacturing an injection needle having a side opening.
Fig. 4A is a plan view schematically showing the injection needle according to one embodiment of the present invention.
Fig. 4B is a side sectional view showing a section A-A in Fig. 4A.
Fig. 5A is a plan view schematically showing the conventional injection needle having a side opening.
Fig. 5B is a side sectional view showing a section B-B in Fig. 5A.
Fig. 6A is a graph showing a change in a piercing resistance value in a forward path when the injection needle is moved back and forth.
Fig. 6B is a graph showing a change in a piercing resistance value in a return path when the injection needle is moved back and forth.
Fig. 7A is a perspective view showing a position of a cross section perpendicular to an axial direction for calculating an outer shape area in an injection needle of 22G according to the present invention.
Fig. 7B is a bent-line graph showing a cross-sectional area change with the axial length from the tip on the horizontal axis and the outer shape area of the cross section perpendicular to the axial direction shown in Fig. 6A on the vertical axis.
Fig. 8A is a perspective view showing a position of a cross section perpendicular to the axial direction for calculating an outer shape area in an injection needle of 30G according to the present invention.
Fig. 8B is a bent-line graph showing a cross-sectional area change with the axial length from the tip on the horizontal axis and the outer shape area of the cross section perpendicular to the axial direction shown in Fig. 8A on the vertical axis.

### DETAILED DESCRIPTION

Next, specific embodiments of the present invention will be described in detail with reference to the drawings. In the drawings, corresponding members having the same function are denoted by the same reference numerals.

### (Injection Needle according to One Embodiment of Present Invention)

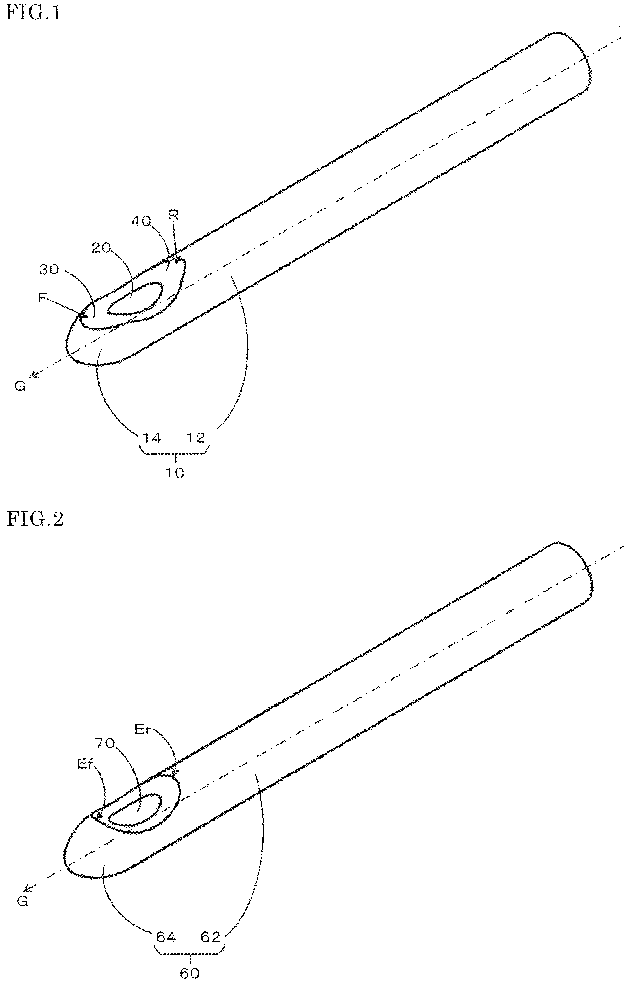

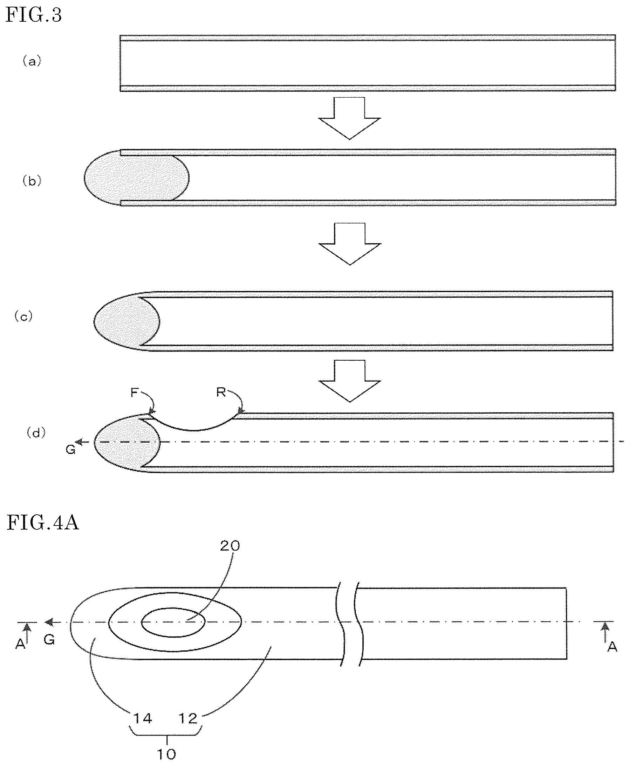

First, an injection needle according to one embodiment of the present invention will be described in comparison with a conventional injection needle having a side opening with reference to Figs. 1 to 3. Fig. 1 is a perspective view schematically showing the injection needle according to one embodiment of the present invention. Fig. 2 is a perspective view schematically showing the conventional injection needle having a side opening. Fig. 3 is a view showing a general method for manufacturing an injection needle having a side opening.

### <Conventional Injection Needle>

For example, in cosmetic surgery in which a medical solution such as hyaluronic acid is administered in the skin, a so-called side hole injection needle 60 having a side surface opening portion 70 as shown in Fig. 2 is used. Such an injection needle 60 includes a hollow cylindrical main body portion 62 and a distal end portion 64 having a curved outer shape and joined so as to close the distal end of the main body portion 62. The side surface opening portion 70 is formed on a side surface in the vicinity of the distal end portion 64 of the injection needle 60. The distal end portion 64 of the injection needle 60 has a spherical shape, and a medical solution is administered from the side surface opening portion 70 formed in the vicinity of the distal end portion 64.

In the case of administering a medicinal solution such as hyaluronic acid, the skin in the vicinity of the region to be administered is punctured in advance with a general injection needle to make a hole, and then the side-hole injection needle 60 is inserted into the hole to administer hyaluronic acid in the skin. In the administration of hyaluronic acid, the injection needle 60 is moved and administered over a wide range in the skin. This makes it possible to expand the skin surface from the intradermal side to extend wrinkles.

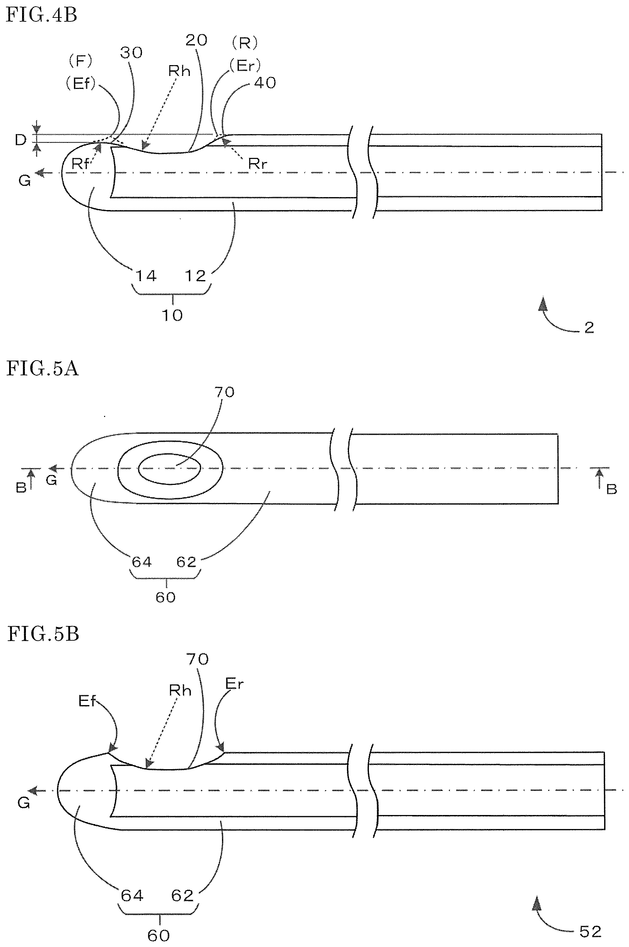

At this time, in order to administer hyaluronic acid over a wide range, the hyaluronic acid is administered to a necessary place by moving or rotating the side-hole injection needle 60 in the skin in the front-back direction. Since the injection needle 60 is moved in a wide range in the skin, there is a risk that intradermal internal bleeding or blood vessel damage may occur. In particular, edge portions Ef and Er exist on the distal end side and the rear end side of the side surface opening portion 70 (see Figs. 2 and 5B), and there is a possibility that the edge portions Ef and Er become resistances and the piercing resistance increases.

### <Method for Manufacturing Injection Needle having Side Surface Opening>

Next, a method for manufacturing the injection needle 60 having such a side surface opening will be described with reference to Fig. 3. First, as shown in (a), a metal circular tube to be the main body portion 62 of the injection needle is prepared. As the material of the metal circular tube, stainless steel such as SUS304 (JIS) and SUS316 (JIS) can be exemplified. As the standard dimensions of the circular tube, 22G: outer diameter 0.7075 mm, thickness 0.105 mm to 30G: outer diameter 0.3105 mm, thickness 0.059 mm can be exemplified, but the standard dimensions are not limited thereto. The length of the metal circular tube is determined according to the length of the injection needle to be manufactured. As the length range, 38 mm to 70 mm can be exemplified, but the length range is not limited thereto.

Next, as shown in (b), a metal lump is welded to one end of the metal circular tube. The metal lump has a curved outer shape and is joined so as to close a tip of the metal lump. The metal lump joined to one end portion of the metal circular tube serves as the distal end portion 64 of the injection needle 60. Then, as shown in (c), polishing is performed so that the main body portion 62 of the injection needle 60 and the distal end portion 64 are smoothly connected.

Next, an elliptical or oval side surface opening portion 70 is formed on the side surface of the injection needle 60 by wire electric discharge machining. More specifically, as shown in (d), wire electric discharge machining is performed such that a region surrounding the side surface opening portion 70 has a substantially arc-shaped concave shape in a side view along the axial direction G in a state where the side surface opening portion 70 is disposed on the upper side. As a result, the side surface opening portion 70 has an elliptical or elliptical shape extending in the axial direction G in a plan view in a state where the side surface opening portion 70 is disposed on the upper side.

The side surface opening portion 70 is preferably formed at a position close to the distal end. However, since an opening cannot be formed when the side surface opening portion 70 reaches a region where the distal end portion 64 exists, it is preferable to form the side surface opening portion 70 as close as possible to the distal end side in a region where the lump of the distal end portion 64 does not exist in the inside.

At this time, as viewed in a side view along the axial direction G in a state where the side surface opening portion 70 is disposed on the upper side, an intersection F between an upper contour line of the main body portion 62 or the distal end portion 64 and a front line segment of a substantially arc-shaped concave shape formed by cutting out by wire electric discharge machining becomes a protruding edge shape, and the edge portion Ef is formed. Similarly, an intersection R between an upper contour line of the main body portion 62 and a substantially arc-shaped concave rear line segment formed by cutting out by wire electric discharge machining becomes a protruding edge shape, and an edge portion Er is formed.

When the injection needle 60 is moved or rotated in the front-back direction in the skin, the edge portions Ef and Er become resistance, and there is a possibility that the piercing resistance increases.

### (Injection Needle according to One Embodiment of Present Invention)

In order to cope with this, in the injection needle 10 according to the present embodiment, a distal end side surface chamfered portion 30 having a gently curved surface is formed at the position corresponding to the edge on the distal end side of the concave shape, and a rear end side surface chamfered portion 40 having a gently curved surface is formed at the position corresponding to the edge on the rear end side of the concave shape.

The injection needle 10 according to the present embodiment also performs manufacturing steps shown in (a) to (d) of Fig. 3. As shown in (a), a metal circular tube to be the main body portion 12 of the injection needle 10 is prepared, and a metal lump is welded to one end of the metal circular tube as shown in (b). As a result, the metal circular tube becomes the main body portion 12 of the injection needle 10, and the welded metal lump becomes the distal end portion 14 of the injection needle 10. Then, as shown in (c), polishing is performed so that the main body portion 12 of the injection needle 10 and the distal end portion 14 are smoothly connected.

Next, as shown in (d), wire electric discharge machining is performed such that a region surrounding the side surface opening portion 20 has a substantially arc-shaped concave shape in a side view along the axial direction G in a state where the side surface opening portion 20 is disposed on the upper side. As a result, the side surface opening portion 70 has an elliptical or elliptical shape extending in the axial direction G in a plan view in a state where the side surface opening portion 20 is disposed on the upper side. In this case, it is preferable to form the side surface opening portion 20 as close as possible to the distal end side in the region where the lump of the distal end portion 14 does not exist inside.

In this state, in a side view along the axial direction G in a state where the side surface opening portion 20 is disposed on the upper side, an intersection F between an upper contour line of the main body portion 12 or the distal end portion 14 and a front line segment of a substantially arc-shaped concave shape formed by cutting out by wire electric discharge machining has a protruding edge shape, and an intersection F between an upper contour line of the main body portion 12 and a rear line segment of a substantially arc-shaped concave shape formed by cutting out by wire electric discharge machining has a protruding edge shape.

In the present embodiment, after the side surface opening portion 20 is formed by wire electric discharge machining as shown in (d), this edge is removed by wire electric discharge machining to form a chamfer portion having a gently curved surface. The distal end side surface chamfered portion 30 having a gently curved surface is formed at a position F which is the edge on the distal end side of the concave shape, and the rear end side surface chamfered portion 40 having a gently curved surface is formed at a position R which is the edge on the rear end side of the concave shape.

More specifically, in the present embodiment, the wire electric discharge machining is performed such that the distal end side surface chamfered portion 30 and the rear end side surface chamfered portion 40 have an arc-shaped convex shape in a side view along the axial direction G in a state where the side surface opening portion 20 is disposed on the upper side. However, the shape is not limited to an arc as long as it has a smooth convex shape.

As described above, the injection needle 10 according to the present embodiment includes: the main body portion 12 having a hollow cylindrical shape; the distal end portion 14 having a curved outer shape, the distal end portion being joined so as to close a distal end of the main body portion 12; and the side surface opening portion 20 opened on a side surface of the main body portion 12, in which the region surrounding the side surface opening portion 20 has a substantially arc-shaped concave shape in a side view along the axial direction G in a state where the side surface opening portion 20 is disposed on the upper side, in which the distal end side surface chamfered portion 30 having a gently curved surface is provided at the position F corresponding to the edge on the distal end side of the concave shape, and in which the rear end side surface chamfered portion 40 having a gently curved surface is provided at the position R corresponding to the edge on the rear end side of the concave shape.

In the injection needle 10 according to the present embodiment, since the distal end side surface chamfered portion 30 having a gently curved surface is formed at the position F which is the edge on the distal end side, and the rear end side surface chamfered portion 40 having a gently curved surface is formed at the position R which is the edge on the rear end side, it is possible to reduce the piercing resistance generated when the injection needle 10 is moved in the skin. By reducing the puncture resistance, the possibility of intradermal internal bleeding and blood vessel damage can be reduced. As a result, it is possible to provide the injection needle 10 capable of reliably reducing the piercing resistance even when the side surface opening portion 20 is provided.

In particular, when the distal end side surface chamfered portion 30 and the rear end side surface chamfered portion 40 have an arc-shaped convex shape in a side view along the axial direction G in a state where the side surface opening portion 20 is disposed on the upper side, the chamfered portion having a gently curved surface can be easily and reliably formed by wire electric discharge machining or the like.

In addition, when the side surface opening portion 20 has an elliptical or oval shape extending in the axial direction G in a plan view in a state where the side surface opening portion 20 is disposed on the upper side, it is possible to reliably administer the medicinal solution including hyaluronic acid to a wide range in the skin.

### (Detailed Shape of Injection Needle according to One Embodiment of Present Invention)

Next, with reference to Figs. 4A to 5B, a more detailed description of the shape of the injection needle according to the present embodiment will be given in comparison with a conventional injection needle. Fig. 4A is a plan view schematically showing an injection needle according to one embodiment of the present invention. Fig. 4B is a side sectional view showing a section A-A in Fig. 4A. Fig. 5A is a plan view schematically showing a conventional injection needle having a side opening. Fig. 5B is a side sectional view showing a section B-B in Fig. 5A.

As shown in Fig. 5B, in the conventional injection needle, the edge portion Ef exists on the distal end side of the side surface opening portion 70, and the edge portion Er exists on the rear end side of the side surface opening portion 70. On the other hand, in the injection needle 10 according to the present embodiment, the distal end side surface chamfered portion 30 having a gently curved surface is formed at the position F which is the edge on the distal end side, and the rear end side surface chamfered portion 40 having a gently curved surface is formed at the position R which is the edge on the rear end side. In Fig. 4A, the outer shapes of the edge portion Ef on the distal end side and the edge portion Er on the rear end side removed by the wire electric discharge machining are indicated by dotted lines.

In a side view along the axial direction G in a state where the side surface opening portion 20 is disposed on the upper side, in the conventional injection needle 60, the edge portion Ef on the distal end side is at substantially the same height position as the upper contour line of the main body portion 12. On the other hand, in the injection needle 10 according to the present embodiment, since the distal end side surface chamfered portion 30 is formed, a distance D in a predetermined height direction exists between the highest position of the distal end side surface chamfered portion 30 and the contour line on the upper side of the main body portion 12 in the side view along the axial direction G in a state where the side surface opening portion 20 is disposed on the upper side. In a case where the conventional edge portion Ef on the distal end side exists, the distance D = 0 in principle.

A larger value of the distance D can be expected to reduce the piercing resistance generated when the injection needle 10 is moved in the skin. In particular, when the injection needle 10 is moved back and forth, it can be expected that the piercing resistance in the forward path is reduced. In addition, since the rear end side surface chamfered portion 40 is smoothly connected to the contour line on the upper side of the main body portion 12 to form a concave shape, it is possible to expect a reduction in piercing resistance in the return path when the injection needle 10 is moved back and forth.

Next, samples of the injection needle 10 having different distances D were experimentally produced, and a test for measuring the piercing resistance value was conducted to examine the relationship between the value of the distance D and the piercing resistance value. In this measurement test, the conventional injection needle 60 (distance D = 0) having the edge portion Ef not having the distal end side surface chamfered portion 30 and injection needles 10 having distances D = 0.04 mm, 0.07 mm, and 0.10 mm were experimentally produced, and the piercing resistance values were measured. An outer diameter of the injection needle 10 was set in a range of 22G or more and 30G or less in consideration of a practical range.

The reason why the range of the distance D is set to the range of 0.04 mm or more and 0.10 mm or less can be described as follows.

The minimum value of the distance D was set to 0.04 mm in consideration of the manufacturing tolerance at the time of forming the distal end side surface chamfered portion 30 and the fact that a certain tendency is stably exhibited even in a repeated test. On the other hand, when the value of the distance D is larger, the effect of reducing the piercing resistance can be expected. However, in consideration of 22G to 30G which are the outer diameters of the injection needle 10 to be actually used, the thickness is 0.059 mm in the case of 30G having the smallest thickness. Therefore, the maximum value of the distance D was set to 0.10 mm in consideration of practical strength.

As shown in Fig. 4A, the region surrounding the side surface opening portion 20 has a substantially arc-shaped concave shape in the side view along the axial direction G in a state where the side surface opening portion 20 is disposed on the upper side. However, as shown in Fig. 4B, R 0.98 mm to R 1.0 mm can be exemplified as the value of the radius Rh of the arc. In addition, in a case where the protruding shape of the distal end side surface chamfered portion 30 and the rear end side surface chamfered portion 40 is an arc shape, R 0.75 mm to R 1.5 mm can be exemplified as a radius Rf and a radius Rr of this arc.

### (Measurement Test of Piercing Resistance Value)

As samples of the injection needle 10 having different distances D, samples having distances D = 0 mm, 0.04 mm, 0.07 mm, and 0.10 mm were experimentally produced for injection needles of 22G and 30G at both ends in a range of 22G or more and 30G or less, and each sample was subjected to 5 measurement tests to obtain an average value.

The puncture target membrane used for the measurement test of the piercing resistance value was made of silicone rubber, and a membrane having as low hardness as possible and a small thickness was used. Specifically, a silicone rubber sheet (hardness 5 (durometer A), thickness: 3 mm, Kyowa Kogyo Co., Ltd.) was used.

In addition, the puncture target membrane was fixed on a measurement jig so that the puncture site was at the center position of the hole having a diameter of 8 mm of the measurement jig. Then, the puncture target membrane was repeatedly punctured twice in a reciprocating manner. The test speed was 20 mm/min. In the first puncture, the second data was adopted because the influence was observed by the opening.

### [Measurement Conditions]

Test apparatus: automatic load tester (MAX-1KN-P-1) manufactured by Japan Instrumentation System Co., Ltd.
Load cell: JCL-M10N (capacity: 10 N)
Measurement jig: MKT-45
Puncture target membrane: silicone rubber sheet (hardness 5 (durometer A), thickness: 3 mm, manufactured by Kyowa Seisakusho Co., Ltd.)
Test speed: 20 mm/min
Test range: about 10 mm from immediately before an injection needle tip comes into contact with the puncture target membrane

### [Measurement Results]

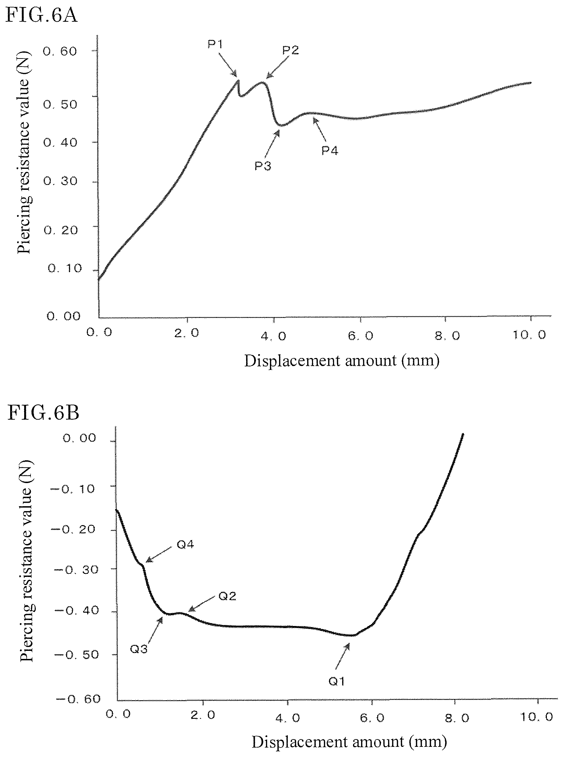

The measurement results are shown in Figs. 6A and 6B. Fig. 6A is a graph showing a change in the piercing resistance value in the forward path when the injection needle is moved back and forth. Fig. 6B is a graph showing a change in the piercing resistance value in the return path when the injection needle is moved back and forth. The horizontal axis of the graph indicates the displacement amount of the distal end of the injection needle. A position immediately before the injection needle tip comes into contact with the puncture target membrane is set to zero. The vertical axis of the graph indicates the measured piercing resistance value (N). The measured piercing resistance value indicates the compression direction as positive.

In any of the experimentally produced injection needles with the distances D of 22G and 30G = 0 mm, 0.04 mm, 0.07 mm, and 0.10 mm, similar tendencies as shown in Figs. 6A and 6B were shown.

### <Forward Path>

In the forward path shown in Fig. 6A, as the injection needle was moved in the piercing direction from the zero position, the piercing resistance value increased, reached the peak P1, and then decreased and increased repeatedly to generate the peak P2, the peak P3, and the peak P4. Here, it is considered that the peak P1 is caused by the deviation of the axis derived from the tension of the puncture target membrane at the time of insertion of the injection needle. Therefore, it is considered that the piercing resistance value of the peak P2 greatly affects the piercing resistance in the forward path. The average value and "Ratio to D = 0" of the piercing resistance values at the measured peaks P2 to P4 are shown in the following table. In "Ratio to D = 0" in the table below, when the piercing resistance value at D = 0 is X and the piercing resistance values at D = 0.04 mm, 0.07 mm, and 0.10 mm are Y, Y/X is expressed in percentage.

**(Table 1)**

| Piercing Resistance Value (Forward Path) | | | | | |
|---|---|---|---|---|---|
| Conditions | | Value of D | | | |
| | | 0 | 0.04 mm | 0.07 mm | 0.10 mm |
| P2 | Average Value (N) | 0.516 | 0.470 | 0.462 | 0.471 |
| | Ratio to D = 0 | - | 91.1% | 89.5% | 91.3% |
| P3 | Average Value (N) | 0.458 | 0.439 | 0.438 | 0.434 |
| | Ratio to D = 0 | - | 96.0% | 95.7% | 94.9% |
| P4 | Average Value (N) | 0.492 | 0.457 | 0.461 | 0.467 |
| | Ratio to D = 0 | - | 92.9% | 93.7% | 94.8% |

It was found that the experimentally produced injection needles with the distances D of 22G and 30G = 0.04 mm, 0.07 mm, and 0.10 mm can reduce the value of the peak P1 in the forward path by about 10% as compared with the injection needle with the distance D = 0.

### <Return Path>

In the return path shown in Fig. 6B, the peak Q1, the peak Q2, the peak Q3, and the peak Q4 were generated by repeating descending and ascending, although not as large as the forward path, as the injection needle was moved from the position pierced in the forward path in the direction of returning the injection needle. Among them, it is considered that the piercing resistance value of the peak Q3 greatly affects the piercing resistance in the forward path. The average value and "Ratio to D = 0" of the measured resistance values at the peaks Q1 to Q4 are shown in the following table.

**(Table 2)**

| Piercing Resistance Value (Return Path) | | | | | |
|---|---|---|---|---|---|
| Conditions | | Value of D | | | |
| | | 0 | 0.04 mm | 0.07 mm | 0.10 mm |
| Q1 | Average Value (N) | -0.484 | -0.439 | -0.439 | -0.437 |
| | Ratio to D = 0 | - | 90.8% | 90.6% | 90.3% |
| Q2 | Average Value (N) | -0.437 | -0.396 | -0.398 | -0.391 |
| | Ratio to D = 0 | - | 90.8% | 91.2% | 89.6% |
| Q3 | Average Value (N) | -0.445 | -0.394 | -0.392 | -0.374 |
| | Ratio to D = 0 | - | 88.5% | 88.1% | 84.1% |
| Q4 | Average Value (N) | -0.312 | -0.301 | -0.302 | -0.292 |
| | Ratio to D = 0 | - | 96.7% | 96.9% | 93.5% |

It was found that the experimentally produced injection needles with the distances D of 30G and 22G = 0.04 mm, 0.07 mm, and 0.10 mm can reduce the value of the peak Q3 in the return path by 10% or more as compared with the injection needle with the distance D = 0.

As described above, when the outer diameter of the main body portion 12 is in the range of 22G or more and 30G or less, and the distance D in the height direction between the highest position of the distal end side surface chamfered portion 30 and the contour line on the upper side of the main body portion 12 is in the range of 0.04 mm or more and 0.10 mm or less in a side view along the axial direction G in a state where the side surface opening portion 20 is disposed on the upper side, it has been found that the piercing resistance value at the second peak P2 in the forward path can be reduced by about 10% as compared with the case of the distance D = 0, and the piercing resistance value at the peak Q3 in the return path can be reduced by 10% or more as compared with the case of the distance D = 0.

Therefore, it has been demonstrated that the injection needle 10 having the distance D in the range of 0.04 mm or more and 0.10 mm or less can reliably reduce the piercing resistance generated when the injection needle 10 is moved in the skin.

### (Outer Area of Cross Section Perpendicular to Axial Direction)

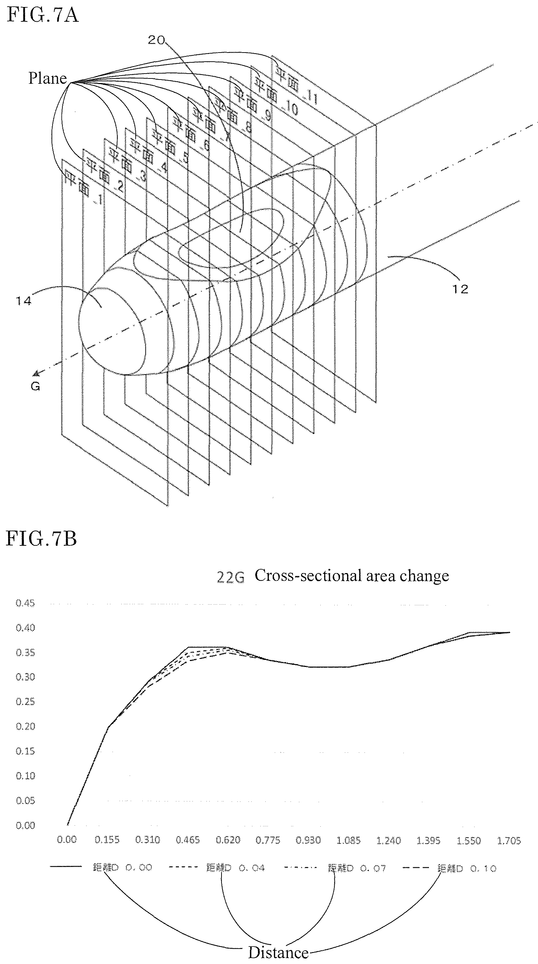

In the above description, the shape of the injection needle 10 according to the present embodiment having the distal end side surface chamfered portion 30 is defined by the distance D in the height direction between the highest position of the distal end side surface chamfered portion 30 and the contour line on the upper side of the main body portion 12, but the present invention is not limited thereto. For example, it is also conceivable to define the outer shape area of the cross section perpendicular to the axial direction G. Next, calculation of the outer shape area of the cross section perpendicular to the axial direction G will be described with reference to Figs. 7A to 8B.

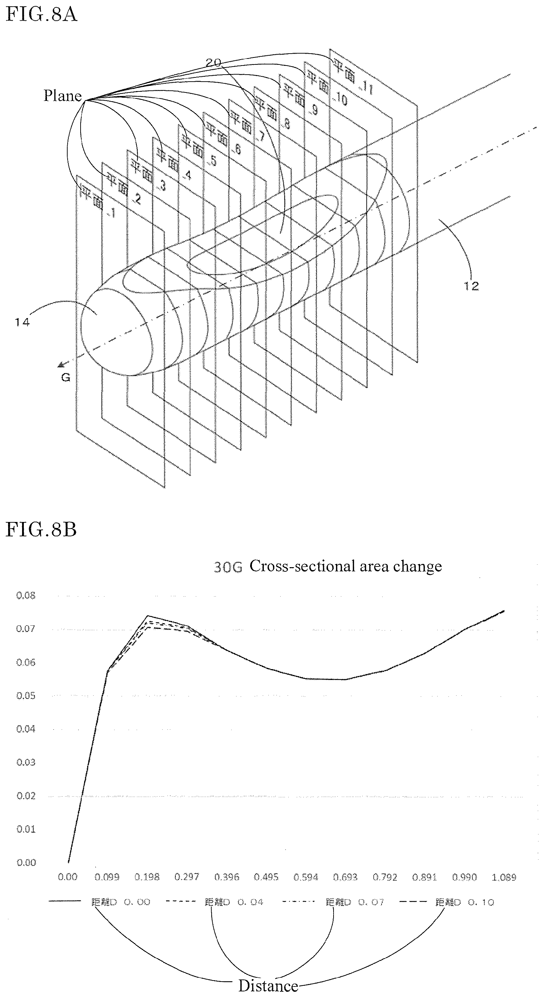

Fig. 7A is a perspective view showing a position of a cross section perpendicular to an axial direction for calculating an outer shape area in the injection needle of 22G according to the present invention. Fig. 7B is a bent-line graph showing a cross-sectional area change with the axial length from the tip on the horizontal axis and the outer shape area of the cross section perpendicular to the axial direction shown in Fig. 6A on the vertical axis. Fig. 8A is a perspective view showing a position of a cross section perpendicular to the axial direction for calculating an outer shape area in an injection needle of 30G according to the present invention. Fig. 8B is a bent-line graph showing a cross-sectional area change with the axial length from the tip on the horizontal axis and the outer shape area of the cross section perpendicular to the axial direction shown in Fig. 8A on the vertical axis.

Fig. 7A shows a plane 1 to a plane 11 forming 11 cross sections of an injection needle 10 of 22G perpendicular to the axial direction. In Fig. 7B, the horizontal axis represents the distance (unit: mm) in the axial direction G from the distal end of the injection needle 10, and the vertical axis represents the outer shape area (unit: mm²) of the cross section perpendicular to the axial direction G. The outer shape area here is an area regarded as solid without subtracting the area of the hollow portion. Fig. 7B is a line graph in which the outer shape areas of the planes 1 to 11 shown in Fig. 7A are plotted and the plotted points are connected by a straight line. The case of the distance D = 0 without the distal end side surface chamfered portion 30 and with the edge portion Ef is indicated by a solid line, the case of the distance D = 0.04 mm with the distal end side surface chamfered portion 30 is indicated by a short dotted line, the case of the distance D = 0.07 mm is indicated by a one-dot chain line, and the case of the distance D = 0.10 mm is indicated by a long dotted line.

With respect to the outer shape area shown in Fig. 7B, the reduction ratio of the outer shape area in the case of including the distal end side surface chamfered portion 30 to the outer shape area in the case of including the edge portion Ef can be expressed by the following formula.

When the outer shape area of the cross section perpendicular to the axial direction G including the edge portion Ef in a case where the edge portion Ef on the distal end side is assumed to be provided is A, and the outer shape area of the cross section perpendicular to the axial direction G in a case where the distal end side surface chamfered portion 30 is provided at the same position in the axial direction G is B, it can be expressed as (A - B)/A. The value of (A - B)/A at the position of the edge portion Ef on the distal end side can be represented by a value in the plane 3 in the following table. On the plane 3, the value of (A - B)/A is in a range of more than 3.0% and less than 7.4%.

With respect to the outer shape area shown in Fig. 7B, the reduction ratio of the outer shape area in the case of including the rear end side surface chamfered portion 40 to the outer shape area in the case of including the edge portion Er can be expressed by the following formula.

When the outer shape area of the cross section perpendicular to the axial direction G including the edge portion Er in a case where the edge portion Er on the distal end side is assumed to be provided is C, and the outer shape area of the cross section perpendicular to the axial direction G in a case where the rear end side surface chamfered portion 40 is provided at the same position in the axial direction G is D, it can be expressed as (C - D)/C. The value of (C - D)/C at the position of the edge portion Er on the rear end side can be represented by a value in the plane 10 in the following table. In the plane 10, the value of (C - D)/C is about 1.8%.

**(Table 3)**

| Reduction Ratio (%) of Outer Area | | | | |
|---|---|---|---|---|
| | Distance (mm) from Tip | Distance D | | |
| | | 0.04 | 0.07 | 0.10 |
| Plane 1 | 0.155 | 0.00% | 0.00% | 0.00% |
| Plane 2 | 0.310 | 0.00% | 1.16% | 4.09% |
| Plane 3 | 0.465 | 3.07% | 4.79% | 7.37% |
| Plane 4 | 0.620 | 0.58% | 1.44% | 2.85% |
| Plane 5 | 0.775 | 0.00% | 0.00% | 0.00% |
| Plane 6 | 0.930 | 0.00% | 0.00% | 0.00% |
| Plane 7 | 1.085 | 0.00% | 0.00% | 0.00% |
| Plane 8 | 1.240 | 0.00% | 0.00% | 0.00% |
| Plane 9 | 1.395 | 0.05% | 0.05% | 0.05% |
| Plane 10 | 1.550 | 1.83% | 1.83% | 1.83% |
| Plane 11 | 1.705 | 0.00% | 0.00% | 0.00% |

The same applies to an injection needle 10 of 30G, and Fig. 8B is a line graph in which the outer shape areas on the planes 1 to 11 shown in Fig. 8A are plotted and the plotted points are connected by a straight line.

With respect to the outer shape area shown in Fig. 8B, the value of (A - B)/A, which is the reduction ratio of the outer shape area in the case of including the distal end side surface chamfered portion 30 to the outer shape area in the case of including the edge portion Ef, can be represented by a value in the plane 2 in the table below. On the plane 2, the value of (A - B)/A is in a range of more than 2.4% and less than 4.8%.

For the outer shape area shown in Fig. 8B, the value of (C - D)/D, which is the reduction ratio of the outer shape area in the case of including the rear end side surface chamfered portion 40 to the outer shape area in the case of including the edge portion Er, can be represented by a value on the plane 11 in the table below. On the plane 11, the value of (C - D)/C is in a range of more than 0.1% and less than 0.6%.

**(Table 4)**

| Reduction Ratio (%) of Outer Area | | | | |
|---|---|---|---|---|
| | Distance (mm) from Tip | Distance D | | |
| | | 0.04 | 0.07 | 0.10 |
| Plane 1 | 0.099 | 0.00% | 0.00% | 0.87% |
| Plane 2 | 0.198 | 2.43% | 3.11% | 4.73% |
| Plane 3 | 0.297 | 0.85% | 1.13% | 2.12% |
| Plane 4 | 0.396 | 0.00% | 0.00% | 0.16% |
| Plane 5 | 0.495 | 0.00% | 0.00% | 0.00% |
| Plane 6 | 0.594 | 0.00% | 0.00% | 0.00% |
| Plane 7 | 0.693 | 0.00% | 0.00% | 0.00% |
| Plane 8 | 0.792 | 0.00% | 0.00% | 0.00% |
| Plane 9 | 0.891 | 0.00% | 0.00% | 0.00% |
| Plane 10 | 0.990 | 0.00% | 0.00% | -0.14% |
| Plane 11 | 1.089 | 0.53% | 0.13% | 0.53% |

The value of (A - B)/A is in a range of more than 3.0% and less than 7.4% in 22G, and a range of more than 2.4% and less than 4.8% in G30G. Therefore, when a range in which both overlap each other is taken, the value of (A - B)/A is in a range of more than 3.0% and less than 4.8%.

Therefore, when the outer diameter of the main body portion 12 is in the range of 22G or more and 30G or less, the outer shape area of the cross section perpendicular to the axial direction G including the edge portion Ef in a case where the edge portion Ef on the distal end side is assumed to be provided is A, and the outer shape area of the cross section perpendicular to the axial direction G in the case of including the distal end side surface chamfered portion 30 at the same position in the axial direction G is B, when the value of (A - B)/A is in the range of more than 3.0% and less than 4.8%, it is possible to reliably reduce the piercing resistance particularly in the forward path, which is generated when the injection needle 10 is moved in the skin.

The value of (C - D)/D is about 1.8% at 22G, and is in a range of more than 0.1% and less than 0.6% at 30G. Therefore, when the value of (C - D)/D is more than at least 0.1%, it can be expected to reduce the piercing resistance particularly in the return path, which is generated when the injection needle 10 is moved in the skin.

Therefore, when the outer diameter of the main body portion is in the range of 22G or more and 30G or less, the outer shape area of the cross section perpendicular to the axial direction G including the edge portion Er in a case where the edge portion Er on the rear end side is assumed to be provided is C, and the outer shape area of the cross section perpendicular to the axial direction G in the case of including the rear end side surface chamfered portion 40 at the same position in the axial direction G is D, when the value of (C - D)/C is in the range of more than 0.1%, it is possible to reliably reduce the piercing resistance particularly in the return path, which is generated when the injection needle 10 is moved in the skin.

Although the embodiment and the aspect of the present invention have been described, the disclosure content may be changed in details of the configuration, and combinations of elements, changes in order, and the like in the embodiment and the aspect can be realized without departing from the scope and spirit of the claimed present invention.

### EXPLANATION OF REFERENCES

- 10: injection needle
- 12: main body portion
- 14: distal end portion
- 20: side surface opening portion
- 30: distal end side surface chamfered portion
- 40: rear end side surface chamfered portion
- 60: injection needle
- 62: main body portion
- 64: distal end portion
- 70: side surface opening portion
- Ef, Er: edge portion
- G: axial direction

## Claims

1. An injection needle comprising:
a main body portion having a hollow cylindrical shape;
a distal end portion having a curved outer shape, the distal end portion being joined so as to close a distal end of the main body portion; and
a side surface opening portion opened on a side surface of the main body portion,
wherein a region surrounding the side surface opening portion has a substantially arc-shaped concave shape in a side view along an axial direction in a state where the side surface opening portion is disposed on an upper side,
wherein a distal end side surface chamfered portion having a gently curved surface is provided at a position corresponding to an edge on a distal end side of the concave shape, and
wherein a rear end side surface chamfered portion having a gently curved surface is provided at a position corresponding to an edge on a rear end side of the concave shape.

2. The injection needle according to claim 1,
wherein an outer diameter of the main body portion is in a range of 22G or more and 30G or less, and
wherein, in a side view along the axial direction in a state where the side surface opening portion is disposed on the upper side, a distance in a height direction between a highest position of the distal end side surface chamfered portion and an upper contour line of the main body portion is in a range of 0.04 mm or more and 0.10 mm or less.

3. The injection needle according to claim 1 or 2,
wherein, when an outer diameter of the main body portion is in a range of 22G or more and 30G or less, an outer shape area of a cross section perpendicular to the axial direction including an edge portion in a case where the edge portion on the distal end side is assumed to be provided is A, and an outer shape area of a cross section perpendicular to the axial direction in a case where the distal end side surface chamfered portion is provided at the same position in the axial direction is B, a value of (A - B)/A is in a range of more than 3.0% and less than 4.8%.

4. The injection needle according to any one of claims 1 to 3,
wherein, when an outer diameter of the main body portion is in a range of 22G or more and 30G or less, an outer shape area of a cross section perpendicular to the axial direction including an edge portion in a case where the edge portion on the rear end side is assumed to be provided is C, and an outer shape area of a cross section perpendicular to the axial direction in a case where the rear end side surface chamfered portion is provided at the same position in the axial direction is D, a value of (C - D)/C is in a range of more than 0.1%.

5. The injection needle according to any one of claims 1 to 4,
wherein, in a side view along the axial direction in a state where the side surface opening portion is disposed on the upper side, the distal end side surface chamfered portion and the rear end side surface chamfered portion have an arc-shaped convex shape.

6. The injection needle according to any one of claims 1 to 5,
wherein, in a plan view in a state where the side surface opening portion is disposed on the upper side, the side surface opening portion has an elliptical or oval shape extending in the axial direction.
